# FASCICULE DE BREVET EUROPEEN

(11) **EP 0 401 140 B1**
(45) Date de publication et mention de la délivrance du brevet: **04.01.1995**
(21) Numéro de dépôt: 90430012.6
(22) Date de dépôt: 28.05.1990
(51) Int. Cl.: A61L 9/01

(54) **Compositions désodorisantes renfermant au moins deux aldéhydes et les produits désodorisants les renfermant**
Desodorierende Zusammensetzungen mit mindestens zwei Aldehyden und diese enthaltende desodorierende Produkte
Deodorant compositions containing at least two aldehydes and the deodorant products contained

(30) Priorité: 29.05.1989 FR 8907279
(43) Date de publication de la demande: 05.12.1990
(73) Titulaire: ROBERTET S.A., 06130 Grasse (FR)
(72) Inventeur: Joulain, Daniel, F-06130 Grasse (FR); Racine, Philippe, F-06130 Grasse (FR)
(74) Mandataire: Rinuy, Santarelli

(56) Documents cités:
- EP-A- 0 132 038
- EP-A- 0 247 946
- FR-A- 1 590 898
- FR-A- 2 599 042
- US-A- 2 875 131

## Description

La présente demande concerne des compositions désodorisantes renfermant au moins deux aldéhydes et les produits désodorisants les renfermant.

La lutte contre les mauvaises odeurs a conduit à utiliser de nombreuses substances de grande variété, par exemple les substances phénoliques, des huiles essentielles, des résines, des aldéhydes ou des cétones, des dérivés alcooliques, des esters ou autres.

EP-A-247 946 décrit des compositions désodorisantes renfermant un certain nombre de produits odoriférants, notamment une composition renfermant parmi une combinaison de 7 principes actifs l'hélional et l'aldéhyde -α- hexylcinnamique dans un rapport volumique de 1 à 31.

On cherche toujours des compositions plus efficaces, désodorisantes et éventuellement d'une odeur agréable.

On a notamment utilisé en mélange avec de nombreux autres produits, divers aldéhydes. Après de longues études, la Demanderesse a découvert que des compositions renfermant un aldéhyde choisi dans une classe, ci après classe A, et un aldéhyde choisi dans une autre classe, ci-après classe B, avaient de remarquables propriétés désodorisantes, nettement supérieures à celles de chacun de ces composés pris individuellement dans certaines proportions.

C'est pourquoi la présente demande a pour objet les compositions désodorisantes caractérisées en ce qu'elles renferment un premier aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique et les aldéhydes bifonctionnels (classe A) et un second aldéhyde choisi parmi les aldéhydes possédant une insaturation non aromatique portée par le carbone en α de la fonction aldéhyde,les aldéhydes possédant une insaturation en α de la fonction aldéhydes conjuguée avec un cycle aromatique et les aldéhydes dont la fonction est portée par un cycle aromatique (classe B), le rapport volumique entre le premier et le second aldéhyde allant de 20/80 à 80/20.

Les aldéhydes aliphatiques acycliques et non terpéniques sont de préférence :
- le décanal,
- l'undécanal,
- le dodécanal,
- l'undécène-10-al,
- le méthyl-2-undécanal,
- le triméthyl-2,6,10 undécène-9 al ("ADOXAL"), et
- le tétraméthyl-2,3,5,5 hexanal.

Les aldéhydes alicycliques non terpéniques sont de préférence :
- le formyl-1 diméthyl-2,4 cyclohexène-2 et le formyl-1 diméthyl-3,5 cyclohexène-4 (TRIPLAL^{R}),
- le formyl-1 triméthyl-2,3,5 cyclohexène-4 et le formyl-1 triméthyl-2,4,6 cyclohexène-3 ("ISOCYCLOCITRAL")
- le (tricyclo[5.2.1.0^{0.6}]décylidène-8)-4 butanal (DUPICAL^{R}),
- le triméthyl-2,6,10 undécène-9 al (ADOXAL^{R}),
- le (méthyl-4-pentène-3 yl)-4 cyclohexène-3 yl carboxaldéhyde,
- le formyl-7 isopropyl-5 méthyl-2 bicyclo 2.2.2. octène-2 (MACEAL^{R}), et
- le formyl-2 diméthyl-8, octahydro-1,2,3,4,5,6,7,8 naphtalène ("ALDEHYDE 111").

Les aldéhydes terpéniques sont de préférence :
- le citronellal, et
- l'aldéhyde campholénique,
Les aldéhydes aliphatiques substitués par un groupe aromatique sont de préférence :
- l'hélional^{R},
- l'aldéhyde cyclamen,
- le lilial,
- le canthoxal^{R},
- l'aldéhyde phénylacétique,
- l'aldéhyde phényl-3 propionique,
- l'aldéhyde hydratropique.

Par "aldéhyde bifonctionnel", l'on entend les aldéhydes possédant par ailleurs une autre fonction comme les fonctions éther-oxyde ou alcool, de préférence :
- des alcoxy-acétaldéhydes,
- des w-hydroxy aldéhydes (hydroxycitronellal, LYRAL^{R}, ...),
- des w-alcoxy-aldéhydes.

Les aldéhydes ci-dessus forment la classe A.

Parmi des aldéhydes de la classe B, les aldéhydes qui possèdent une insaturation de type non aromatique portée par le carbone en position α de la fonction aldéhyde sont de préférence :
- le citral (néral et géranial),
- le myrténal,
- l'aldéhyde périlla,
- les furyl-2 carboxaldéhydes diversement substitués.

Les aldéhydes qui possèdent une insaturation éthylénique en α, elle-même conjuguée avec un cycle aromatique sont de préférence :
- l'aldéhyde cinnamique,
- l'aldéhyde α-amylcinnamique ("JASMONAL"),
- l'aldéhyde α-hexylcinnamique.

Les aldéhydes portés par un cycle aromatique, par ailleurs diversement substitués, sont de préférence :
- le benzaldéhyde,
- l'aldéhyde anisique,
- l'héliotropine,
- l'aldéhyde vératrique,
- la vanilline,
- l'isovanilline, et
- l'éthylvanilline.

Les compositions désodorisantes selon la présente invention renferment un aldéhyde de chacune des classes ci-dessus et peuvent bien évidemment renfermer trois ou plusieurs aldéhydes pour autant que chacune des deux classes ci-dessus soit représentée.

Dans des conditions préférentielles de mise en oeuvre de l'invention ci-dessus décrite, le premier aldéhyde est choisi de préférence parmi les aldéhydes aliphatiques acycliques et non terpéniques, les aldéhydes alicycliques non terpéniques, les aldéhydes terpéniques et les aldéhydes aliphatiques substitués par un groupe aromatique, le second aldéhyde est choisi de préférence parmi les aldéhydes qui possèdent une insaturation de type non aromatique portée par le carbone α.

Tout particulièrement, le premier aldéhyde est choisi parmi les aldéhydes aliphatiques acycliques et non terpéniques et le second parmi ceux ci-dessus désignés.

Des couples d'aldéhydes particulièrement intéressants sont notamment les couples suivants :
- le dodécanal et le myrténal,
- le dodécanal et le citral,
- l'adoxal et l'aldéhyde Perilla,
- le triplal et le citral,
- le macéal et le citral,
- l'adoxal et le myrténal,
- le décanal et le citral,
- l'undécène-10-al et le myrténal,
- ainsi que les couples de produits cités dans les exemples.

Les aldhéhydes des classes A et B peuvent se trouver en proportions relatives l'une par rapport à l'autre et de préférence en proportions de 80/20 à 20/80 notamment en proportions de 50/50.

Les compositions désodorisantes ci-dessus, en plus de leurs remarquables propriétés désodorisantes, possèdent en outre par elles-mêmes des propriétés odoriférantes capables de remplacer les mauvaises odeurs par leur propre odeur.

Des compositions préférées renferment en outre un agent masquant.

Ces compositions peuvent donc comporter au moins un agent odoriférant d'odeur agréable tels que ceux habituellement utilisés en parfumerie comme des alcools, huiles essentielles, substances phénoliques, esters.

Ces compositions désodorisantes trouvent leur application dans toutes les conditions où lutte contre les mauvaises odeurs est recherchée, quelle que soit leur origine.

Les compositions ci-dessus décrites peuvent renfermer en outre des produits spécifiques, par exemple des bactéricides.

Ces compositions désodorisantes sont avantageusement formulées selon les techniques habituelles.

C'est pourquoi la présente demande a également pour objet les produits désodorisants caractérisés en ce qu'ils renferment les compositions ci-dessus décrites.

Ces produits peuvent se présenter sous forme de pulvérisateurs aérosols, supports solides imprégnés, liquides, crèmes, poudres, etc. Ils peuvent être réalisés selon les méthodes connues de l'homme de l'art.

### Partie expérimentale

Les exemples qui suivent illustrent la présente invention.

L'effet sur les mauvaises odeurs des compositions selon la présente invention a été évalué à l'aide des dispositifs suivants :

On dirige de l'azote à l'aide d'un raccord en Y vers deux débitmètres munis d'une valve permettant de régler le débit dans chacune des branches du dispositif. Ce gaz alimente deux cristallisoirs identiques en verre d'un volume de 25 litres contenant respectivement un mélange malodorant et le même mélange additionné des produits à tester, destinés à neutraliser ce mélange malodorant, par deux courants gazeux de même débit.

Chacun des deux cristallisoirs est muni d'un couvercle amovible en verre et est relié également à un récipient d'olfaction par des tubulures en verre. Un bouchon d'arrêt permet, soit de laisser passer le courant gazeux du cristallisoir vers le récipient d'olfaction, soit d'isoler ce dernier.

Les récipients d'olfaction sont également coiffés d'un couvercle en verre.

Avant chaque évaluation des produits à tester, on prépare deux échantillons identiques d'une mauvaise odeur standard que l'on place dans chacun des cristallisoirs.

On place alors 2 g de produits à tester sur de l'ouate de cellulose que l'on dépose dans l'un des deux cristallisoirs. Ceux-ci sont alors fermés par leur couvercle et recouverts d'un tissu opaque afin que les évaluateurs réalisent le test en aveugle. Le produit à tester est en solution 10 mM. Lorsque l'on teste un produit seul, on place sur l'ouate 2 g de solution de ce produit et pour un mélange de deux produits, 1 g de solution de chacun d'eux.

Le débit du gaz vecteur est réglé à 2 litres/minute dans chacune des deux branches du dispositif pour une durée de 3 minutes.

On ferme alors les bouchons de manière à isoler les récipients d'olfaction du reste du montage. Les évaluateurs viennent alors sentir dans les deux récipients d'olfaction, soit immédiatement à la fin des 3 minutes, soit 5 minutes après.

On demande aux évaluateurs de juger les différences d'intensité et de tonalité d'odeurs entre les deux récipients d'olfaction.

Les évaluateurs sont des personnes entraînées à percevoir les différences de tonalité et d'intensité des odeurs.

Les essais ont été conduits avec un panel de personnes et leur réponse notée et codée comme suit :
- - :: pas de différence entre les deux flacons ou réponse erronée, quand on demande où se trouvent les produits en cours de test
- ± :: différence de tonalité entre les deux récipients mais les odeurs sont perçues comme étant malodorantes
- + :: atténuation sensible de la mauvaise odeur dans le récipient où se trouve(nt) le(s) produit(s) en cours de test
- ++ :: disparition totale de la mauvaise odeur dans le récipient où se trouve(nt) le(s) produit(s) en cours de test.

On a testé individuellement des aldéhydes choisis dans la classe A ou B. Les résultats obtenus sont les suivants :

**Tableau 1**

| Classe A | Classe B | Effet à < 1 mn. | Effet à 5 mn. |
|---|---|---|---|
| | citral | - | - |
| hélional | | - | + |
| dodécanal | | + | + |
| | jasmonal | - | + |
| citronellal | | + | + |

On a ensuite testé les couples d'aldéhydes appartenant à une même classe. Les résultats obtenus sont les suivants :

**Tableau 2**

| | Couple | Couple |
|---|---|---|
| Classe A | dodécanal/hélional | |
| Classe B | | citral |
| | | jasmonal |
| Effet à < 1 mn. | - | - |
| Effet à 5 mn. | ++ | + |

On a enfin testé les couples d'aldéhydes appartenant aux classes A et B. Les résultats obtenus sont les suivants :

**Tableau 3**

| | Couple | Couple | Couple |
|---|---|---|---|
| Classe A | hélional | hélional | citronellal |
| Classe B | citral | jasmonal | jasmonal |
| Effet à < 1 mn. | + | - | - |
| Effet à 5 mn. | + | ++ | ++ |

En conclusion, l'effet neutralisant le plus net et le plus fort, non seulement immédiatement mais également après un délai de 5 minutes, est obtenu lorsque l'on utilise des couples d'aldéhydes appartenant à la fois aux deux classes A et B.

N.B. : Le dodécanal a été testé en solution 2,5 mM et non 10 mM comme les autres produits, de manière à ne pas saturer l'enceinte par son odeur.

### Préparation d'une mauvaise odeur standard

On verse sur un tampon d'ouate de cellulose vierge, 1 g d'une solution à 1000 ppm de propylamine et de 1 g d'une solution à 100 ppm de mercapto-3 méthyl-3 butanol-1.

### 1. Aérosol désodorisant d'atmosphère

On a réalisé un aérosol répondant à la composition suivante (en volumes) :

| | |
|---|---|
| - citronellal | 1 % |
| - jasmonal | 1 % |
| - éthanol à 99 % | 3 % |
| - Fréon 11 | 57 % |
| - Fréon 12 | 38 % |

### 2. Aérosol désodorisant d'atmosphère

On a réalisé un aérosol répondant à la composition suivante (en volumes) :

| | |
|---|---|
| - citral | 1,5 % |
| - jasmonal | 0,5 % |
| - éthanol à 99 % | 3,0 % |
| - Fréon 11 | 57,0 % |
| - Fréon 12 | 38,0 % |

### 3. Désodorisant et désinfectant liquide pour sols :

On a réalisé un désodorisant et désinfectant liquide répondant à la composition suivante (en volumes):

| | |
|---|---|
| - nonylphénol éthoxylé à 10 moles d'oxyde d'éthylène | 7,0 % |
| - chlorure d'alkylméthylbenzylammonium | 1,5 % |
| - citral | 0,5 % |
| - hélional | 0,5 % |
| - eau permutée | 90,5 % |

### 4. Désodorisant et désinfectant liquide pour sols :

- On a réalisé un désodorisant et désinfectant liquide répondant à la composition suivante (en volumes):

| | |
|---|---|
| - nonylphénol éthoxylé à 10 moles d'oxyde d'éthylène | 7,0 % |
| - chlorure d'alkylméthylbenzylammonium | 1,5 % |
| - dodécanal | 0,2 % |
| - hélional | 0,8 % |
| - eau permutée | 90,5 % |

## Revendications (Revendications pour l'(les) Etat(s) contractant(s) suivant(s): AT, BE, CH, LI, DE, DK, FR, GB, GR, IT, LU, NL, SE)

1. Compositions désodorisantes, caractérisées en ce qu'elles renferment un premier aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels et un second aldéhyde choisi parmi les aldéhydes possédant une insaturation non aromatique portée par le carbone en α de la fonction d'aldéhyde, les aldéhydes possédant l'insaturation en α de la fonction aldéhyde conjuguée avec un cycle aromatique et les aldéhydes dont la fonction est portée par un cycle aromatique, le rapport volumique entre le premier et le second aldéhyde allant de 20/80 à 80/20.

2. Compositions désodorisantes selon la revendication 1, caractérisées en ce que le premier aldéhyde est choisi parmi les aldéhydes aliphatiques acycliques et non terpéniques, les aldéhydes alicycliques non terpéniques, les aldéhydes terpéniques et les aldéhydes aliphatiques substitués par un groupe aromatique, le second aldéhyde est choisi parmi les aldéhydes qui possèdent une insaturation de type non aromatique portée par le carbone α.

3. Compositions désodorisantes selon la revendication 1 ou 2, caractérisées en ce que le premier aldéhyde est choisi parmi las aldéhydes aliphatiques acycliques et non terpéniques et le second parmi les aldéhydes qui possèdent une insaturation de type non aromatique portée par le carbone α.

4. Compositions désodorisantes selon l'une quelconque des revendications 1 à 3, caractérisées en ce qu'elles renferment de l'hélional et du citral, de l'hélional et du jasmonal, ou du citronellal et du jasmonal.

5. Compositions désodorisantes selon l'une quelconque des revendications 1 à 4, caractérisées en ce qu'elles renferment en outre un agent masquant.

6. Compositions désodorisantes selon l'une quelconque des revendications 1 à 5, caractérisées en ce qu'elles renferment en outre un bactéricide.

7. Produits désodorisants caractérisés en ce qu'ils renferment une composition selon l'une quelconque des revendications 1 à 6.

## Revendications (Revendications pour l'(les) Etat(s) contractant(s) suivant(s): ES)

1. Procédé de préparation de compositions désodorisantes, caractérisé en ce que l'on mélange un premier aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels avec un second aldéhyde choisi parmi les aldéhydes possédant une insaturation non aromatique portée par le carbone en α de la fonction aldéhyde, les aldéhydes possédant l'insaturation en α de la fonction aldéhyde conjuguée avec un cycle aromatique et les aldéhydes dont la fonction est portée par un cycle aromatique, le rapport volumique entre le premier et le second aldéhyde allant de 20/80 à 80/20.

2. Procédé selon la revendication 1, caractérisé en ce que le premier aldéhyde est choisi parmi les aldéhydes aliphatiques acycliques et non terpéniques, les aldéhydes alicycliques non terpéniques, les aldéhydes terpéniques et les aldéhydes aliphatiques substitués par un groupe aromatique, le second aldéhyde est choisi parmi les aldéhydes qui possèdent une insaturation de type non aromatique portée par le carbone α.

3. Procédé selon la revendication 1 ou 2, caractérisé en ce que le premier aldéhyde est choisi parmi les aldéhydes aliphatiques acycliques et non terpéniques et le second parmi les aldéhydes qui possèdent une insaturation de type non aromatique portée par le carbone α.

4. Procédé selon l'une quelconque des revendications 1 à 3, caractérisé en ce que l'on mélange de l'hélional et du citral, de l'hélional et du jasmonal, ou du citronellal et du jasmonal.

5. Procédé selon l'une quelconque des revendications 1 à 4, caractérisé en ce que l'on mélange en outre un agent masquant.

6. Procédé selon l'une quelconque des revendications 1 à 5, caractérisé en ce que l'on mélange en outre un bactéricide.

7. Procédé de préparation d'un produit désodorisant caractérisé en ce que l'on prépare un produit désodorisant renfermant une composition selon l'une quelconque des revendications 1 à 6.

8. Compositions désodorisantes, caractérisées en ce qu'elles renferment un premier aldéhyde choisi parmi les aldéhydes aliphatiques acycliques et non terpéniques, les aldéhydes alicycliques non terpéniques, les aldéhydes terpéniques, les aldéhydes aliphatiques substitués par un groupe aromatique, les aldéhydes bifonctionnels et un second aldéhyde choisi parmi les aldéhydes possédant une insaturation non aromatique portée par le carbone en α de la fonction aldéhyde, les aldéhydes possédant l'insaturation en α de la fonction aldéhyde conjuguée avec un cycle aromatique et les aldéhydes dont la fonction est portée par un cycle aromatique, le rapport volumique entre le premier et le second aldéhyde allant de 20/80 à 80/20.

9. Compositions désodorisantes selon la revendication 8, caractérisées en ce que le premier aldéhyde est choisi parmi les aldéhydes aliphatiques acycliques et non terpéniques, les aldéhydes alicycliques non terpéniques, les aldéhydes terpéniques et les aldéhydes aliphatiques substitués par un groupe aromatique, le second aldéhyde est choisi parmi les aldéhydes qui possèdent une insaturation de type non aromatique portée par le carbone α.

10. Compositions désodorisantes selon l'une quelconque des revendications 8 et 9, caractérisées en ce que l'on mélange de l'hélional et du citral, de l'hélional et du jasmonal, ou du citronellal et du jasmonal.

## Claims (Claims for the following Contracting State(s): AT, BE, CH, LI, DE, DK, FR, GB, GR, IT, LU, NL, SE)

1. Deodorant compositions, characterized in that they contain a first aldehyde chosen from the acyclic and non-terpenic aliphatic aldehydes, the non-terpenic alicyclic aldehydes, the terpenic aldehydes, the aliphatic aldehydes substituted by an aromatic group, the bifunctional aldehydes, and a second aldehyde chosen from the aldehydes possessing a non-aromatic unsaturation carried by the carbon in alpha position of the aldehyde function, the aldehydes possessing the unsaturation in alpha position of the aldehyde function conjugated with an aromatic ring and the aldehydes of which the function is carried by an aromatic ring, the quantitative ratio between the first and the second aldehyde ranging from 20/80 to 80/20.

2. Deodorant compositions according to claim 1, characterized in that the first aldehyde is chosen from the acyclic and non-terpenic aliphatic aldehydes, the non-terpenic alicyclic aldehydes, the terpenic aldehydes and the aliphatic aldehydes substituted by an aromatic group, the second aldehyde is chosen from the aldehydes which possess an unsaturation of non-aromatic type carried by the alpha carbon.

3. Deodorant compositions according to claim 1 or 2, characterized in that the first aldehyde is chosen from the acyclic and non-terpenic aliphatic aldehydes and the second from the aldehydes which possess an unsaturation of non-aromatic type carried by the alpha carbon.

4. Deodorant compositions according to any one of claims 1 to 3, characterized in that they contain helional and citral, helional and jasmonal, or citronellal and jasmonal.

5. Deodorant compositions according to any one of claims 1 to 4, characterized in that they contain in addition a masking agent.

6. Deodorant compositions according to any one of claims 1 to 5, characterized in that they contain in addition a bactericide.

7. Deodorant products, characterized in that they contain a composition according to any one of claims 1 to 6.

## Claims (Claims for the following Contracting State(s): ES)

1. Preparation process for deodorant compositions, characterized in that a first aldehyde chosen from the acyclic and non-terpenic aliphatic aldehydes, the non-terpenic alicyclic aldehydes, the terpenic aldehydes, the aliphatic aldehydes substituted by an aromatic group, the bifunctional aldehydes, is mixed with a second aldehyde chosen from the aldehydes possessing a non-aromatic unsaturation carried by the carbon in alpha position of the aldehyde function, the aldehydes possessing the unsaturation in alpha position of the aldehyde function conjugated with an aromatic ring and the aldehydes of which the function is carried by an aromatic ring, the quantitative ratio between the first and the second aldehyde ranging from 20/80 to 80/20.

2. Process according to claim 1, characterized in that the first aldehyde is chosen from the acyclic and non-terpenic aliphatic aldehydes, the non-terpenic alicyclic aldehydes, the terpenic aldehydes and the aliphatic aldehydes substituted by an aromatic group, the second aldehyde is chosen from the aldehydes which possess an unsaturation of non-aromatic type carried by the alpha carbon.

3. Process according to claim 1 or 2, characterized in that the first aldehyde is chosen from the acyclic and non-terpenic aliphatic aldehydes and the second from the aldehydes which possess an unsaturation of non-aromatic type carried by the alpha carbon.

4. Process according to any one of claims 1 to 3, characterized in that helional and citral, helional and jasmonal, or citronellal and jasmonal are mixed together.

5. Process according to any one of claims 1 to 4, characterized in that in addition a masking agent is mixed in.

6. Process according to any one of claims 1 to 5, characterized in that in addition a bactericide is mixed in.

7. Preparation process for a deodorant product, characterized in that a deodorant product is prepared containing a composition according to any one of claims 1 to 6.

8. Deodorant compositions, characterized in that they contain a first aldehyde chosen from the acyclic and non-terpenic aliphatic aldehydes, the non-terpenic alicyclic aldehydes, the terpenic aldehydes, the aliphatic aldehydes substituted by an aromatic group, the bifunctional aldehydes, and a second aldehyde chosen from the aldehydes possessing a non-aromatic unsaturation carried by the carbon in alpha position of the aldehyde function, the aldehydes possessing the unsaturation in alpha position of the aldehyde function conjugated with an aromatic ring and the aldehydes of which the function is carried by an aromatic ring, the quantitative ratio between the first and the second aldehyde ranging from 20/80 to 80/20.

9. Deodorant compositions according to claim 8, characterized in that the first aldehyde is chosen from the acyclic and non-terpenic aliphatic aldehydes, the non-terpenic alicyclic aldehydes, the terpenic aldehydes and the aliphatic aldehydes substituted by an aromatic group, the second aldehyde is chosen from the aldehydes which possess an unsaturation of non-aromatic type carried by the alpha carbon.

10. Deodorant compositions according to any one of claims 8 and 9, characterized in that helional and citral, helional and jasmonal, or citronellal and jasmonal are mixed together.

## Patentansprüche (Patentansprüche für folgende(n) Vertragsstaat(en): AT, BE, CH, LI, DE, DK, FR, GB, GR, IT, LU, NL, SE)

1. Desodorisierende Zusammensetzungen, dadurch gekennzeichnet, daß sie umfassen: einen ersten Aldehyd, ausgewählt aus den aliphatischen acyclischen und nicht-terpenischen Aldehyden, den alicyclischen nicht-terpenischen Aldehyden, den terpenischen Aldehyden, den aliphatischen Aldehyden, substituiert durch eine aromatische Gruppe, und den bifunktionellen Aldehyden, und einen zweiten Aldehyd, ausgewählt aus den Aldehyden, die eine nicht-aromatische Unsättigung aufweisen, welche vom α-Kohlenstoff der Aldehydfunktion getragen wird, den Aldehyden, die eine Unsättigung in Stellung α zur Aldehydfunktion, konjugiert mit einem aromatischen Ring aufweisen, und den Aldehyden, deren Funktion von einem aromatischen Ring getragen wird, wobei das Volumsverhältnis zwischen dem ersten und dem zweiten Aldehyd von 20/80 bis 80/20 beträgt.

2. Desodorisierende Zusammensetzungen nach Anspruch 1, dadurch gekennzeichnet, daß der erste Aldehyd ausgewählt ist aus den aliphatischen acyclischen und nicht-terpenischen Aldehyden, den alicyclischen nicht-terpenischen Aldehyden, den terpenischen Aldehyden und den aliphatischen Aldehyden, substituiert durch eine aromatische Gruppe, und der zweite Aldehyd ausgewählt ist aus den Aldehyden, die eine Unsättigung vom nicht-aromatischen Typ aufweisen, welche vom α-Kohlenstoff getragen wird.

3. Desodorisierende Zusammensetzungen nach Anspruch 1 oder 2, dadurch gekennzeichnet, daß der erste Aldehyd ausgewählt ist aus den aliphatischen acyclischen und nicht-terpenischen Aldehyden, und der zweite Aldehyd ausgewählt ist aus den Aldehyden, die eine Unsättigung vom nicht-aromatischen Typ aufweisen, welche vom α-Kohlenstoff getragen wird.

4. Desodorisierende Zusammensetzungen nach einem der Ansprüche 1 bis 3, dadurch gekennzeichnet, daß sie Helional und Citral, Helional und Jasmonal oder Citronellal und Jasmonal umfassen.

5. Desodorisierende Zusammensetzungen nach einem der Ansprüche 1 bis 4, dadurch gekennzeichnet, daß sie ferner ein Maskierungsmittel umfassen.

6. Desodorisierende Zusammensetzungen nach einem der Ansprüche 1 bis 5, dadurch gekennzeichnet, daß sie ferner ein Bakterizid umfassen.

7. Desodorisierende Produkte, dadurch gekennzeichnet, daß sie eine Zusammensetzung nach einem der Ansprüche 1 bis 6 umfassen.

## Patentansprüche (Patentansprüche für folgende(n) Vertragsstaat(en): ES)

1. Verfahren zur Herstellung desodorisierender Zusammensetzungen, dadurch gekennzeichnet, daß gemischt wird: ein erster Aldehyd, ausgewählt aus den aliphatischen acyclischen und nicht-terpenischen Aldehyden, den alicyclischen nicht-terpenischen Aldehyden, den terpenischen Aldehyden, den aliphatischen Aldehyden, substituiert durch eine aromatische Gruppe, und den bifunktionellen Aldehyden, mit einem zweiten Aldehyd, ausgewählt aus den Aldehyden, die eine nicht-aromatische Unsättigung aufweisen, welche vom α-Kohlenstoff der Aldehydfunktion getragen wird, den Aldehyden, die eine Unsättigung in Stellung α zur Aldehydfunktion, konjugiert mit einem aromatischen Ring aufweisen, und den Aldehyden, deren Funktion von einem aromatischen Ring getragen wird, wobei das Volumsverhältnis zwischen dem ersten und dem zweiten Aldehyd von 20/80 bis 80/20 beträgt.

2. Verfahren nach Anspruch 1, dadurch gekennzeichnet, daß der erste Aldehyd ausgewählt ist aus den aliphatischen acyclischen und nicht-terpenischen Aldehyden, den alicyclischen nicht-terpenischen Aldehyden, den terpenischen Aldehyden und den aliphatischen Aldehyden, substituiert durch eine aromatische Gruppe, und der zweite Aldehyd ausgewählt ist aus den Aldehyden, die eine Unsättigung vom nicht-aromatischen Typ aufweisen, welche vom α-Kohlenstoff getragen wird.

3. Verfahren nach Anspruch 1 oder 2, dadurch gekennzeichnet, daß der erste Aldehyd ausgewählt ist aus den aliphatischen acyclischen und nicht-terpenischen Aldehyden, und der zweite Aldehyd ausgewählt ist aus den Aldehyden, die eine Unsättigung vom nicht-aromatischen Typ aufweisen, welche vom α-Kohlenstoff getragen wird.

4. Verfahren nach einem der Ansprüche 1 bis 3, dadurch gekennzeichnet, daß Helional und Citral, Helional und Jasmonal oder Citronellal und Jasmonal gemischt werden.

5. Verfahren nach einem der Ansprüche 1 bis 4, dadurch gekennzeichnet, daß ferner ein Maskierungsmittel beigemischt wird.

6. Verfahren nach einem der Ansprüche 1 bis 5, dadurch gekennzeichnet, daß ferner ein Bakterizid beigemischt wird.

7. Verfahren zur Herstellung eines desodorisierenden Produkts, dadurch gekennzeichnet, daß ein desodorisierendes Produkt, das eine Zusammensetzung nach einem der Ansprüche 1 bis 6 umfaßt, hergestellt wird.

8. Desodorisierende Zusammensetzungen, dadurch gekennzeichnet, daß sie umfassen: einen ersten Aldehyd, ausgewählt aus den aliphatischen acyclischen und nicht-terpenischen Aldehyden, den alicyclischen nicht-terpenischen Aldehyden, den terpenischen Aldehyden, den aliphatischen Aldehyden, substituiert durch eine aromatische Gruppe, und den bifunktionellen Aldehyden, und einen zweiten Aldehyd, ausgewählt aus den Aldehyden, die eine nichtaromatische Unsättigung aufweisen, welche vom α-Kohlenstoff der Aldehydfunktion getragen wird, den Aldehyden, die eine Unsättigung in Stellung α zur Aldehydfunktion konjugiert mit einem aromatischen Ring aufweisen, und den Aldehyden, deren Funktion von einem aromatischen Ring getragen wird, wobei das Volumsverhältnis zwischen dem ersten und dem zweiten Aldehyd von 20/80 bis 80/20 beträgt.

9. Desodorisierende Zusammensetzungen nach Anspruch 8, dadurch gekennzeichnet, daß der erste Aldehyd ausgewählt ist aus den aliphatischen acyclischen und nicht-terpenischen Aldehyden, den alicyclischen nicht-terpenischen Aldehyden, den terpenischen Aldehyden und den aliphatischen Aldehyden, substituiert durch eine aromatische Gruppe, und der zweite Aldehyd ausgewählt ist aus den Aldehyden, die eine Unsättigung vom nicht-aromatischen Typ aufweisen, welche vom α-Kohlenstoff getragen wird.

10. Desodorisierende Zusammensetzungen nach Anspruch 8 oder 9, dadurch gekennzeichnet, daß Helional und Citral, Helional und Jasmonal oder Citronellal und Jasmonal gemischt werden.
